# NEW EUROPEAN PATENT SPECIFICATION

(11) **EP 0 163 230 B2**
(45) Date of publication and mention of the opposition decision: **06.03.1996**
(45) Mention of the grant of the patent: 29.03.1989
(21) Application number: 85106173.9
(22) Date of filing: 20.05.1985
(51) Int. Cl.: C07B 39/00, C07C 25/13

(54) **Process for producing aromatic chlorine compounds**
Verfahren zur Herstellung von chlorierten aromatischen Verbindungen
Procédé pour la préparation de composés aromatiques chlorés

(30) Priority: 22.05.1984 JP 101670/84
(43) Date of publication of application: 04.12.1985
(73) Proprietor: ASAHI GLASS COMPANY LTD., Chiyoda-ku, Tokyo (JP)
(72) Inventor: Kumai, Seisaku, Shinagawa-ku Tokyo (JP); Seki, Ryuji, Yokohama-shi Kanagawa-ken (JP); Matsuo, Masashi Shiinokidai Haitsu 3-304, Yokohama-shi Kanagawa-ken (JP)
(74) Representative: Wächtershäuser, Günter, Prof. Dr.

(56) References cited:
- US-A- 4 424 396
- US-A- 4 470 930
- GMELINS Handbuch der Anorganischen Chemie, "Phosphor", Teil C, Verlag Chemie GmbH, 1965
- Zhurnal Obshchei Khimil, vol. 31, No.4, pages 1222-1226. April 1961
- Chemical Abstracts, 1961, 24605i -24606 a, b, e.
- Chemical Abstracts, vol. 52, 19987 h,i
- Chemical Abstracts, vol. 59, 1507 b, c
- Chemical Abstracts vol. 79, 31599 n (1973)
- Anorganische Chemie, K L Hofmann, 1963, p. 274
- BIOS final report No. 986/Part 1, page 151
- Ullmanns Encyklopädie der technischen Chemie, Band 5, 1954, page 466
- Chemie der Elemente, N N Greenwood, 1. Auflage, 1988, Seite 566, Tabelle 11.6

## Description

The present invention relates to a discontinuous process for producing aromatic chlorine compounds from aromatic nitro compounds according to the preamble of claim 1 such process is known from US-A-4424396. In the known process PCI₅ is used as the chlorinating agent.

Further, as a process for producing an aromatic chlorine compound such as 2,6-dichlorofluorobenzene from an aromatic nitro compound such as 3-chloro-2-fluoronitrobenzene, the following process is known.

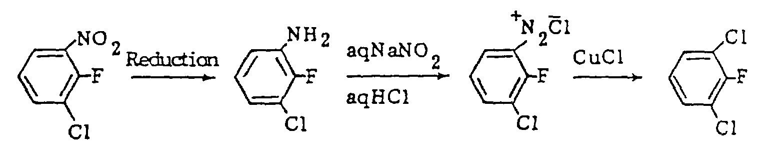

However, the intermediate product i.e. the diazonium salt, has an extremely high toxicity, and the adverse effects to the workers is a serious problem. Further, the diazonium salt is unstable, and readily undergoes decomposition at room temperature, whereby it is impossible to obtain the desired compound in good yield. Moreover, the above-mentioned invention involves a number of steps, each step having a low volume efficiency, and large amount of acidic waste liquid forms, treatment of which is costly. Thus, the conventional process has several drawbacks.

The present inventors have conducted extensive researches with an aim to develop a process for producing an aromatic chlorine compound from an aromatic nitro compound in an industrially advantageous manner, on the basis of their knowledge that an aromatic chlorine compound can be obtained in a single step reaction without bringing about an environemntal problem by directly substituting chlorine for the nitro group bonded directly to a carbon atom of the aromatic ring. As a result, it has been found that this substitution reaction of chlorine for the nitro group is affected by the substituents adjacent to the nitro group, i.e. the chlorine substitution hardly takes place when both sides of the nitro group are occupied by hydrogen atoms, and when at least one of the adjacent positions is occupied by a halogen atom, the reactivity remarkably improves. From a further research, the present inventors have found another interesting fact that by conducting the substitution of chlorine for the nitro group in the substantial absence of water, the yield can be remarkably improved. The present invention has been accomplished on the basis of these discoveries.

Namely, the present invention provides a process for producing batchwise an aromatic chlorine compound of the following formula II, which comprises substituting chlorine for the nitro group of an aromatic nitro compound of the following formula I by reaction with chlorine gas in the substantial absence of water: 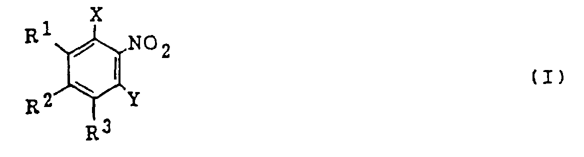 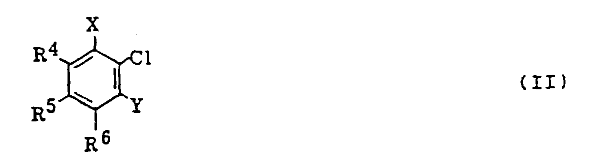 wherein each of X and Y is Cl, Br, F or H, provided that X and Y are not simultaneously H, and each of R¹, R, R³, R⁴, R⁵ and R⁶ is Cl, Br, F, NO₂, CN, CF₃, OCF₃ or H, characterized in that chlorine gas is added to the reaction sytem in an amount of 0.2 to 10 times the theoretical amount required for the substitution of chlorine for the nitro group and the substitution is conducted in the presence of a dehydrating agent.

Now, the present invention will be described in detail with reference to the preferred embodiments.

The aromatic nitro compound in the present invention is represented by the above formula I and is a compound having at least one nitro group and at least one halogen atom such as Cl, Br or F, simultaneously. Further, there is at least one pair of adjacent carbon atoms in the aromatic ring on which the nitro group and the halogen atom are attached. Namely, in the formula I, at least one of X and Y adjacent to the nitro group is a halogen atom. If X and Y are H simultaneously, the chlorination of the nitro group sandwiched by X and Y hardly takes place. On the other hand, the resulting aromatic chlorine compound is represented by the above formula II. In the formulas I and II, X and Y respectively represent Cl, Br, F or H, and include all combinations thereof except for the combination of X and Y being H simultaneously. R¹, R and R³ respectively represent Cl, Br, F, NO₂, CN, CF₃ or H, and include combinations of 1 to 3 kinds thereof. Likewise, R⁴, R⁵ and R⁶ respectively represent Cl, Br, F, NO₂, CN, CF₃ or H, and include combinations of 1 to 3 kinds thereof.

The substitution reaction of chlorine for the nitro group is conducted in a liquid phase or vapor phase system. In the liquid phase system, the reaction may be conducted in the presence or absence of a solvent. When a solvent is employed, it is preferred to use a halogen solvent such as a chlorinated paraffin, a chlorotrifluoroethylene low molecular weight polymer or tetrabromoethane. The chlorine gas is used in an amount of from 0.2 to 10 times, preferably from 1.0 to 5 times, the theoretical amount required for the substitution of chlorine for the nitro group in the aromatic nitro compound, to be substituted by chlorine. The reaction is suitably conducted at a temperature of from 150 to 350°C, under a reaction pressure of from atmospheric pressure to 100 kg/cm for a reaction time of from 2 to 30 hours.

In the chlorine substitution reaction of the present invention, the formation of NO₂Cl has been confirmed. This is believed to be attributable to the chlorination reaction of the NO₂ group. This NO₂Cl can readily be separated from the desired compound by alkali washing. In the process of the present invention, water forms as a by-product. Although the mechanism of the formation is not clearly understood, it is believed that water forms as a by-product by the reaction of the above-mentioned NO₂Cl with HCI which forms as a by-product by the chlorination of the ring. Anyway, the by-product water reacts with the starting material, whereby the yield of the desired compound will decrease, and if water is incorporated into the starting material chlorine, the corrosive property remarkably increases. Thus, it is important to conduct the substitution reaction of chlorine for the nitro group, in the substantial absence of water. For instance, in the case where the starting material aromatic nitro compound is 3-chloro-2-fluoronitrobenzene, an undesirable side reaction takes place whereby the nitro compound reacts with water formed as a by-product, to form HF, as shown below.

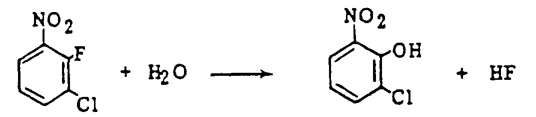

HF, particularly water-containing HF, is likewise highly corrosive, and it not only corrodes the material of the apparatus, but also causes a side reaction with the corroded metal material serving as a negative catalyst. Therefore, in the case where a starting material which is likely to form HF, is to be used, it is preferred to conduct the chlorine substitution reaction for the nitro group in the presence of a HF-binding agent. As preferred HF-binding agents, there may be mentioned CaCl₂, SiO₂, NaF and KF.

In order to maintain the chlorine substitution reaction for the nitro group in the substantial absence of water, it is effective to conduct the reaction in the presence of a dehydrating agent. As the dehydrating agent, an inorganic dehydrating agent which is inert to the starting material and the reaction product and which has a substantial water-binding property, is preferred. Namely, it is preferred to use a dehydrating agent which is capable of reacting with water at a temperature of at least 150°C to form a hydrate, or has a strong water-absorbing property, and which has a property not to readily release the bound water. Preferred specific examples include SbCl₅, SbF₅, SbBr₃, SnCl₄, Tacl₅, BF₃, CaCl₂, PCI₅, SiO₂, P₂O₅ and synthetic zeolite. They may be used alone or in combination as a mixture. CaCl₂ or SiO₂ is useful also as a HF-binding agent.

The type of the HF-binding agent or the dehydrating agent to give good yield of the desired compound, varies depending upon the desired compound, and may be appropriately selected. The HF-binding agent is used in an amount of from 0.01 to 1 mol per mol of the starting material aromatic nitro compound. Likewise, the dehydrating agent is used preferably in an amount of from 0.01 to 1 mol.

According to the process of the present invention, aromatic chlorine compounds such as 2,6-dichlorofluorobenzene, 3-chloro-4-fluorobenzenetrifluoride, 3,4,5-trichlorobenzotrifluoride and 1-chloro-2,3,4-trifluorobenzene, which are useful as the starting materials for intermediates of medicines or agricultural chemicals, can be obtained advantageously in an industrial manner.

Now, the present invention will be described with reference to Examples.

### Example 1

Into a 200 ml Hastelloy® C autoclave, 140 g (0.80 mol) of 3-chloro-2-fluoronitrobenzene and 16.6 g (0.080 mol) of phosphorus pentachloride were charged, and chorine gas was introduced at a rate of 6.3 g/hr at 180°C for 15 hours.

The reaction solution was analyzed, whereby the conversion of the starting material was 85% and the selectivity for 2,6-dichlorofluorobenzene was 96%. Further, the corrosion rate in this reaction was 0.05 mm/ year. In this Example and the following Examples and Comparative Examples, the corrosion rate was measured as follows.

### Corrosion test method

A test piece of Hastelloy® (30 × 15 × 3 mm) or glass (5 φ × 50 mm) was placed in a reactor, and the chlorination reaction was conducted. The weight reduction of the test piece in this reaction was converted to the depth of erosion per year (8000 hours), which represents the corrosion rate.

Corrosion rate (mm/year) = Δ W × 8000/S × P × T
Δ W: weight reduction (mg)
S: surfce area of the test piece (mm)
P: density of the test piece (mg/mm³)
T: reaction time (hr)

### Example 2

To the charge of Example 1, 7 g of fine silica (SiO₂ in a fine particulate form) was added, and the reaction was conducted in the same manner as in Example 1. The reaction solution was analyzed, whereby the conversion of the starting material was 83%, and the selectivity for 2,6-dichlorofluorobenzene was 95%. The corrosion rate of the glass in the reaction was less than 0.01 mm/year.

### Example 3

Into a 200 ml glass reactor, 140 g (0.80 mol) of 3-chloro-2-fluoronitrobenzene and 8.9 g (0.080 mol) of anhydrous calcium chloride were charged, and chlorine gas was introduced at a rate of 6.3 g/hr at 180°C for 15 hours. The reaction solution was analyzed, whereby the conversion of the starting material was 82%, and the selectivity for 2,6-dichlorofluorobenzene was 98%. The corrosion rate of glass in this reaction was 0.04 mm/year.

### Example 4

Into a 200 ml glass reactor, 100 g (0.45 mol) of 3-chloro-4,6-dinitrofluorobenzene and 5.0 g (0.045 mol) of anhydrous calcium chloride were charged, and chlorine gas was introduced at a rate of 7 g/hr at 200°C for 15 hours. The reaction solution was analyzed, whereby the conversion of the starting material was 90%, and the selectivity for 1-fluoro-2,4,5-trichlorobenzene was 97%. The corrosion rate of the glass in this reaction was 0.06 mm/year.

### Example 5

Into a 200 ml Hastelloy® reactor, 140 g (0.80 mol) of 4-chloro-2-fluoronitrobenzene and 16.6 g (0.080 mol) of phosphorus pentachloride were charged, and chlorine gas was introduced at a rate of 6.3 g/hr at 180°C for 20 hours. The reaction solution was analyzed, whereby the conversion of the starting material was 92%, and the selectivity for 2,5-dichlorofluorobenzene was 88%. The corrosion rate was glass in this reaction was 0.08 mm/year.

### Example 6

Into a 200 ml glass reactor, 200 g of 4-fluoro-3-nitrobenzotrifluoride and 5.3 g of anhydrous calcium chloride were charged, and chlorine gas was introduced at a rate of 27 g/hr at 180°C under a pressure of 7 kg/cm for 6 hours, whereby the conversion of the starting material was 94.6%, and the selectivity for 3-chloro-4-fluorobenzotrifluoride was 98.5%. Then, the reaction solution was washed with water and then distilled to obtain 149 g of 3-chloro-4-fluorobenzotrifluoride having a purity of 99.8%.

### Example 7

Into a 200 ml Hastelloy® C autoclave, 150 g of 2,3,4-trifluoronitrobenzene and 17.5 g of phosphorus pentachloride were charged and heated to about 190°C. While maintaining the pressure at a level of from 5 to 7 kg/cm, chlorine gas was introduced at a rate of about 25 g/hr for 4 hours. The reaction solution was analyzed, whereby the conversion of the starting material was 93%, and the selectivity for 1-chloro-2,3,4-trifluorobenzene was 88%.

### Example 8

Into a 200 ml Hastelloy® C autoclave, 150 g of 3,4-dichloro-5-nitrobenzotrifluoride and 16 g of phosphorus pentachloride were charged and heated to about 190°C. While maintaining the pressure at a level of about 5 kg/cm, chlorine gas was introduced at a rate of about 25 g/hr for 4 hours. The reaction solution was analyzed, whereby the conversion of the starting material was 92%, and the selectivity for 3,4,5-trichlorobenzotrifluoride was 89%.

### Comparative Example 1

The reaction was conducted in the same manner as in Example 1 except that no phosphorus pentachloride was used, whereby the selectivity for 2,6-dichlorofluorobenzene was 65% at a conversion of the starting material of 85%. The corrosion rate of the glass in this reaction was 20 mm/year.

### Comparative Example 2

The reaction was conducted in the same manner as in Example 3 except that no anhydrous calcium chloride was used, whereby the selectivity for 2,6-dichlorofluorobenzene was 79% at a conversion of the starting material of 82%. The corrosion rate of the glass in this reaction was 18 mm/year.

### Comparative Example 3

The reaction was conducted in the same manner as in Example 7 except that no phosphorus pentachloride was used, whereby the selectivity for 1-chloro-2,3,4-trifluorobenzene was 60% at a conversion of the starting material of 93%.

## Claims

1. A discontinuous process for producing batchwise an aromatic chlorine compound of the following formula II, which comprises substituting chlorine for the nitro group of an aromatic nitro compound of the following formula I by reaction with chlorine gas in the substantial absence of water 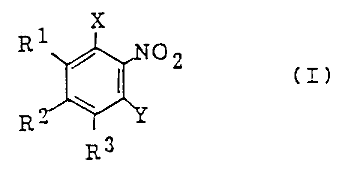 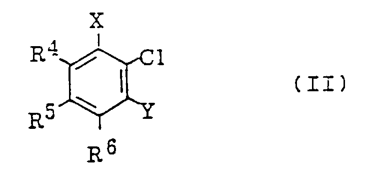 wherein each of X and Y is Cl, Br, F or H, provided that X and Y are not simultaneously H, and each of R¹, R, R³, R⁴, R⁵ and R⁶ is Cl, Br, F, NO₂, CN, CF₃, OCF₃ or H, **characterized** in that chlorine gas is added to the reaction system in an amount of 0.2 to 10 times the theoretical amount required for the substitution of chlorine for the nitro group and the substitution is conducted in the presence of a dehydrating agent.

2. The process according to Claim 1, wherein the dehydrating agent is an inorganic dehydrating agent which is inert to the starting materials and the reaction product and has a great water binding property.

3. The process according to Claim 2, wherein the dehydrating agent is at least one member selected from the group consisting of CaCl₂, PCl₅ and SiO₂.

## Patentansprüche

1. Nicht-kontinuierliches Verfahren zur chargenweisen Herstellung einer aromatischen Chlorverbindung der folgenden Formel II, umfassend eine Substitution der Nitrogruppe einer aromatischen Nitroverbindung der folgenden Formel I mit Chlor durch Umsetzung mit Chlorgas im wesentlichen in Abwesenheit von Wasser 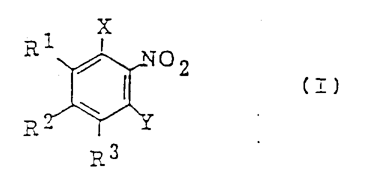 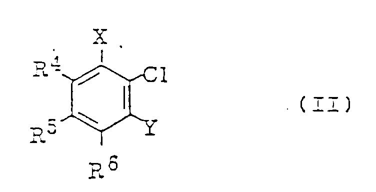 wobei X und Y jeweils Cl, Br, F oder H ist, vorrausgesetzt, daß X und Y nicht gleichzeitig H sind, und R¹, R, R³, R⁴, R⁵ und R⁶ jeweils Cl, Br, F, NO₂, CN, CF₃, OCF₃ oder H ist, dadurch gekennzeichnet, daß Chlorgas zu dem Reaktionssystem in einer Menge der 0,2 bis 10-fachen theoretischen Menge zugegeben wird, die für die Substitution der Nitrogruppe durch Chlor erforderlich ist, und daß die Substitution in Gegenwart eines Entwässerungsmittels durchgeführt wird.

2. Verfahren gemäß Anspruch 1, wobei das Entwässerungsmittel ein anorganisches Entwässerungsmittel ist, welches gegenüber den Ausgangsmaterialien und dem Reaktionsprodukt inert ist und ein großes Wasserbindungsvermögen aufweist.

3. Verfahren gemäß Anspruch 2, wobei das Entwässerungsmittel mindestens ein Element aus der Menge, bestehend aus CaCl₂, PCl₅ und SiO₂, ist.

## Revendications

1. Procédé de fabrication discontinue d'un composé aromatique chloré représenté par la formule (II) suivante, qui comprend le remplacement, par le chlore, du groupe nitro d'un composé aromatique nitré représenté par la formule (I) suivante, par réaction avec du chlore gazeux, sensiblement en l'absence d'eau : 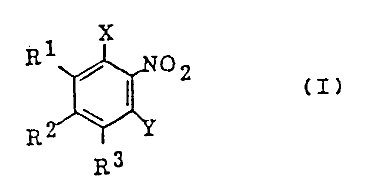 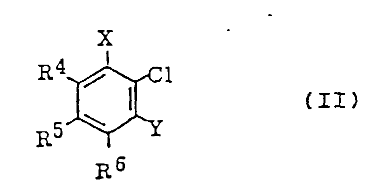 où :
- X et Y représentent chacun Cl, Br, F ou H, à la condition que X et Y ne représentent pas simultanément H ; et
- R¹, R, R³, R⁴, R⁵ et R⁶ représentent chacun Cl, Br, F, NO₂, CN, CF₃, OCF₃ ou H,
caractérisé par le fait qu'on ajoute du chlore gazeux au système réactionnel en une quantité de 0,2 à 10 fois la quantité théorique requise pour le remplacement du groupe nitro par le chlore et que le remplacement est effectué en présence d'un agent déshydratant.

2. Procédé selon la revendication 1, dans lequel l'agent déshydratant est un agent déshydratant minéral, qui est inerte vis-à-vis des matières de départ et du produit de la réaction et qui présente la propriété de lier fortement l'eau.

3. Procédé selon la revendication 2, dans lequel l'agent déshydratant est au moins un élément choisi dans le groupe constitué par CaCl₂, PCl₅ et SiO₂.
